# EUROPEAN PATENT SPECIFICATION

(11) **EP 1 054 651 B1**
(45) Date of publication and mention of the grant of the patent: **23.04.2003**
(21) Application number: 99922015.5
(22) Date of filing: 19.05.1999
(51) Int. Cl.: A61F 7/12

(54) **ENDOMETRIAL ABLATION METHOD AND APPARATUS**
GERÄT ZUR ABLATION DES ENDOMETRIUMS
TECHNIQUE ET APPAREIL POUR ABLATION DE L'ENDOMETRE

(30) Priority: 22.05.1998 CA 2232726
(43) Date of publication of application: 29.11.2000
(73) Proprietor: Elliott, James, Richmond, British Columbia V7A 4V4 (CA); Yackel, Douglass, Surrey, British Columbia V4A 4A7 (CA); Luo, Qiyi, Richmond, British Columbia V7C 4H4 (CA)
(72) Inventor: Elliott, James, Richmond, British Columbia V7A 4V4 (CA); Yackel, Douglass, Surrey, British Columbia V4A 4A7 (CA); Luo, Qiyi, Richmond, British Columbia V7C 4H4 (CA)
(74) Representative: Allard, Susan Joyce
(86) International application number: CA9900476
(87) International publication number: WO99060960

(56) References cited:
- WO-A-94/07445
- WO-A-94/21202
- US-A- 3 924 628
- US-A- 5 084 044
- US-A- 5 449 380
- US-A- 5 720 762

## Description

### FIELD OF THE INVENTION

The present invention relates to an apparatus and method for thermally ablating the endometrial tissue lining the uterine cavity. More specifically, (he apparatus and method of the present invention uses a pre-shaped balloon and ensures proper deployment of the balloon, through mechanical means, to conform to the shape of the uterus. In addition the method of thermal ablation is significantly changed when compared to known intrauterine thermal ablation techniques.

### BACKGROUND OF THE INVENTION

A tissue ablator is a device used to cauterize, or induce necrosis, of living tissue. Intrauterine tissue ablators are useful for treating menorrhagia and metrorrhagia, excessive bleeding conditions sometimes associated with pain.

Ablation is usually accomplished by thermal or cryogenic treatments. One thermal treatment involves treatment of the tissue with a laser or electro-cautery device. A physician using this procedure for uterine ablation must "paint" the intrauterine surface with the laser beam or electro-cautery probe, making it difficult to uniformly treat the entire intrauterine area. Incomplete treatment can result in continued bleeding discomfort. Also inherent in the laser or electro-cautery techniques is the risk that an area of the tissue surface will be punctured from the prolonged exposure to the beam or probe.

Another method for tissue ablation involves an inflatable bladder which is inserted into the organ and inflated with a thermal or cryogenic substance. The inflated bladder contacts the surrounding tissue and the extreme temperature of the thermal or cryogenic substance in the balloon causes the tissue to necrose.

One such device intended for intrauterine cauterization is disclosed in U.S. Pat. No.'s 4,949,718 and 5,105,808. An inflatable bladder located at the end of a catheter is inserted into the uterus. A liquid medium is used to fill the bladder, causing it to inflate inside the uterus. Heating coils located inside the bladder heat the medium to temperatures of 60.0°C to 101.7°C (140°F to 215°F) (temperatures known to induce necrosis of endometrial lining) for a period of 4-12 minutes. This device suffers from the disadvantage of using a substantially spherical bladder, whereas the uterus is bicornate in shape. This introduces the potential for leaving areas untreated. The long treatment time can increase patient and physician discomfort. Moreover, the heating element is located inside the body during treatment. The heating coil reaches temperatures much higher than the fluid temperature and its placement in the bladder creates the risk that the patient may be burned. The heating and temperature probe are located centrally and remote from the endometrial lining resulting in temperatures at the lining are less than at the probe by unknown amounts.

Another thermal ablation device intehded for intrauterine cauterization is disclosed in U.S. Pat. No. 5,449,380. An inflatable bladder located at the end of a catheter is inserted into the uterus. The device contains an inflation means for circulation of an inflation fluid through the catheter and the balloon, and a heating means for heating the inflation fluid to temperatures of 87.8°C to 104.4°C (190°F to 220°F) The balloon is mechanically shaped to approximate the shape of the organ. This device suffers from the disadvantage of lack of control of heat transfer to the tissue to be necrosed due to the introduction of a circulating fluid where temperature is controlled outside of the bladder. This introduces opportunities for fluid short-circuits, improper mixing, potential dead zones where fluid temperatures are not sufficient to effectively ablate tissue and non uniform heating leading to untreated areas. Also, this device does not disclose the selection and use of one of two differently shaped bladders.

Another thermal ablation device intended for endometrial ablation is disclosed U.S. Pat. No. 5,084,044. A thermally conductive inflatable member is inserted into the uterus. The inflatable member is filled with fluid that could be heated prior to, during passage to 15 the inflatable member or once inside the inflatable member. The inflation fluid is heated to temperatures of about 50.0°C to 99.4°C (122°F to 211°F) and is claimed to urge the inflatable member into the expanded position and into intimate contact with the tissue of the cavity. A predetermined time for treatment is selected to be in the range between 30 seconds and 10 minutes.

### SUMMARY OF THE INVENTION

The present invention consists of an apparatus which thermally ablates tissue by introducing heated fluid through pre-shaped balloon which is inserted into the uterus on the tip of a catheter. The pre-shape is designed for two sizes nulliparuos and parous. In addition the balloon is mechanically deployed in a 3 dimensional fashion to ensure the conforming of the pre-shaped balloon to the uterus and effectively treating the entire uterus.

The apparatus of the present invention may be used according to a method of thermal ablation utilizing higher temperatures (above 104.4°C (220°F)) than previously used in prior art and for a shorter period of time. Furthermore the treatment may be performed twice to provide for even greater efficacy of the ablation technique.

The apparatus of the present invention provides advantages over prior art to achieve more uniform and complete endometrial lining contact at monitored, higher temperatures for a more efficacious thermal ablation treatment. Specifically the accumulative advantages are provided by:
a) Provision of two balloon sizes - one for nulliparous and one for parous uteri.
b) Pre-shaped balloons conforming to the actual bicornate and shallow depth of the uterus.
c) Positional indicators to assure proper insertion.
d) An optional mechanically aided 3-D deployment of the balloon.
f) Higher than previous art temperatures - at known and uniform values.
e) Shorter than previous art treatment times with superior ergonometrics to ease physician stress.
g) Dual treatment procedure to take advantage of improved necrosis of the endometrial lining.
h) Use of biocompatible, non-allergenic material for balloon construction.

In addition, the front end reusable equipment has been designed to provide improved variable control at low costs to the end user.

### DESCRIPTION OF THE DRAWINGS

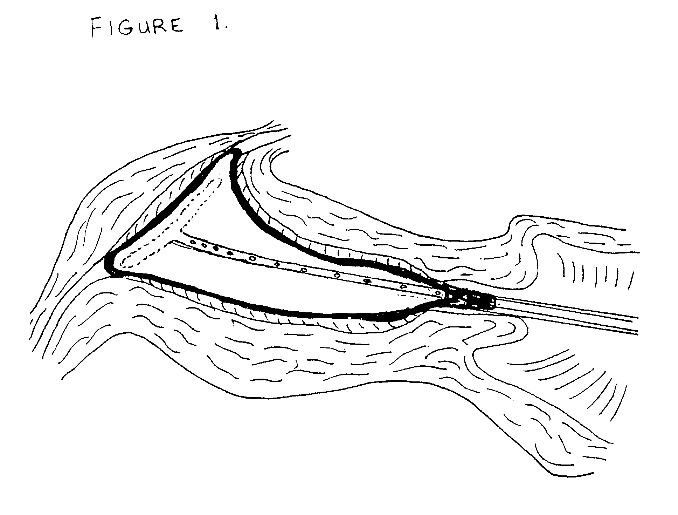

FIG. 1 is a 3-dimensional view of the inside of the uterus showing a pre-formed 3-dimensionally mechanically shaped balloon according to the present invention.
FIG. 2 is a schematic for the entire endometrial ablation apparatus.

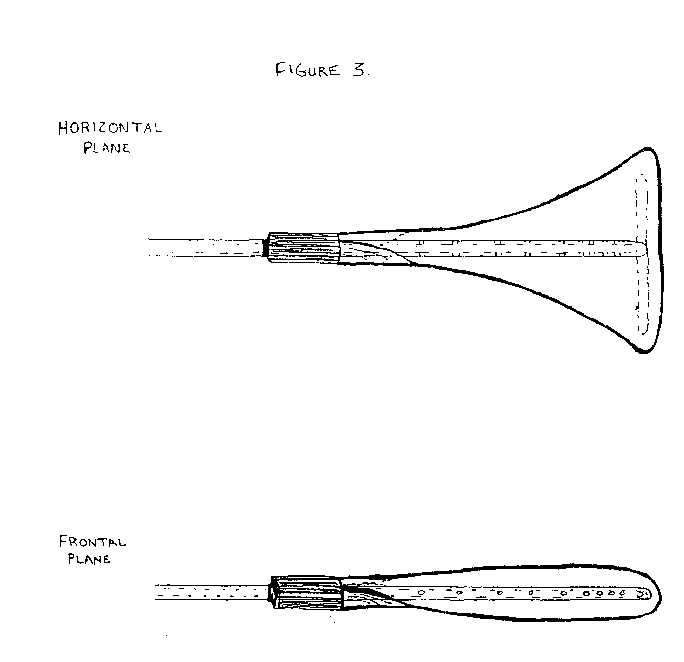

FIG. 3 is a horizontal and frontal plane view of the endometrial ablator part (II) without the optional retractable sleeve.

### DETAILED DESCRIPTION

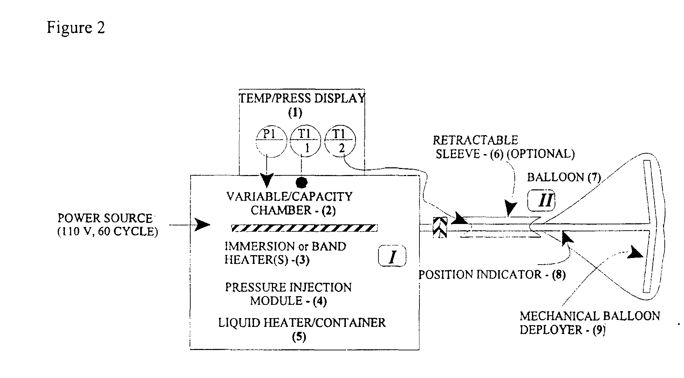

Referring to figure 2, the heater/injector part (**I**) contains: a variable capacity chamber (**2**) that provides reliable temperature pressure monitoring, vapor venting, vapor separation, and sufficient volume to prevent temperature decline through heat loss; a built-in temperature/pressure display (**1**) to provide a display **T1-2** of temperature of the hot liquid - (The liquid will be solutions that will allow liquid temperatures in excess of 104.4°C (220°F) without risk of boiling. Examples of such solutions are mixtures of n-Butyl alcohol or similar even-numbered Carbon, non-toxic alcohols, glycerol, glycerol/water mixtures, and mineral oil) - in the endometrial ablator part (**II**) via a thermocouple located inside the balloon (**7**). In addition, pressure **P1** and temperature **T1-1** inside the heater/injector part (**I**) is monitored; a pressure injection module (**4**) ergonometrically designed to provide easy and efficient control of liquid injection into the balloon, pressure regulation and liquid withdrawal from the balloon; a liquid capacity chamber (**5**) of sufficient volume to assure deliverance of up to 100 ml of temperature controlled liquid to the balloon for a dual ablation procedure; a specification standard immersion heater(s) or specification standard band heater(s) (**3**) fitted into or around the liquid heater/container (**5**) and is equipped with a regulator for temperature control connected by a standard cord plugged into a standard 110 V/ 60 cycle outlet and; stopcock valves for liquid charging, venting, draining and control of liquid movements into and out of the balloon, plus quick connect coupling to avoid introduction of air to purged part (**II**).

Referring to figure 2, an endometrial ablator part (**II**) according to the present invention is comprised of : a pre-shaped inflatable balloon (**7**) which is sealed around the distal end of a catheter - (The balloon material is: flexible (softness, strength, workability similar to Latex) and; chemically resistant to n-Butyl alcohol or similar even-numbered Carbon, non-toxic alcohols, non-toxic alcohol-Glycerol solutions, non-toxic glycerol-water solutions, non-toxic mineral oil solutions at temperatures greater than 104.4°C (220°F); compatible with molding techniques; compatible with non-toxic, biocompatible coatings to prevent sticking to uterine cavities.); a catheter that delivers inflation liquid and couples as a position indicator (**8**) for both balloon depth and rotational orientation; an optional retractable sleeve (**6**) providing - a pre-sheathing of the balloon for ease of part (**II**) delivery, a restraint for an optional solid mechanical 3-dimensional balloon deployer (**9**) with positional memory and, an insulation shield to protect the cervix, cervical glands and uterus; an optional 3-dimensional mechanical balloon deployer (**9**) that provides assurance that the balloon will properly deploy within the uterine chamber and conform to the shape of the uterus - (The optional mechanical balloon deployer is manufactured of appropriate material that is non-toxic and super-elastic and strong enough to reliably deploy the pre-shaped balloon inside the uterine chamber. In addition the mechanical balloon deployer will allow easy withdrawal of the endometrial ablator part (**II**) post treatment.) - and; a standard quick connect coupling at the distal end to allow for quick connection to part (**I**).

Use of the preferred embodiment will next be described.
STEP 1: Standard pre-sizing of uterus determines depth that device will be inserted.
STEP 2: Charge liquid to part **(II)** with valve to part (**I**) closed.
STEP 3: Heat liquid in the liquid/container (**5**) via the immersion heater (**3**) to desired temperature (>104.4°C (220°F))
STEP 4: Air is purged from part (**II**) by partially filling the system with saline solution. This partial filling is performed prior to insertion of the device into the uterus, with the retractable sleeve (**6**)(if applicable) held in the distal position to prevent the balloon from inflating.
STEP 5: Connect part (**I**) to part (**II**).
STEP 6: Insert part **(II)** into uterus using position indicator (**8**) to ensure proper depth and rotational orientation of the pre-shaped balloon.
STEP 7: Pull back retractable sleeve (if applicable) (**6**).
STEP 8: Start timing and flush hot inflation liquid into part (**II**) for 30 - 90 seconds.
STEP 9: Monitor balloon temperature and when temperature is below 104.4°C (220°F) withdraw inflation liquid from part (**II**).
STEP 10: Repeat STEPS 8 and 9.
STEP 11: Drain liquid into disposable liquid container, or if part (**I**) is disposable discard part (**I**) and part (**II**). If part (**II**) is not disposable disconnect after draining and discard part (**II**).

## Claims

1. An apparatus for thermally ablating tissue in a uterus, comprising:
(a) a catheter having a proximal end and a distal end;
(b) two-sizes of pre-shaped inflatable balloons, (for parous and nulliparous uteri) individually attached to the distal end of the catheter which will conform to the shape of the uterus;
(c) a balloon deployment system connected to the distal end of the catheter that is deployed via mechanical and/or hydraulic means that in turn engages the balloon and deploys the pre-shaped balloon;
(d) a balloon inflation means for pushing inflation fluid through the capacitive element, the catheter and into the balloon;
(e) a balloon deflation means for withdrawing inflation fluid from the balloon, the catheter and from the capacitive element;
(f) a heating means for heating the inflation fluid to an elevated temperature of above 104.4°C (220°F)
(g) a chamber to provide a capacitive element allowing reliable pressure monitoring, vapor venting, vapor separation and sufficient volume to prevent temperature decline through heat loss; and
wherein the catheter has a longitudinal axis and wherein mechanical balloon deployment means includes deployment members that have two positions -a contracted position in which the deployment members are substantially parallel to the longitudinal axis and an extended position in which the members extend perpendicular to the longitudinal axis of the catheter and attached at the end of the catheter, thereby ensuring proper balloon deployment and conformation to the inside of the uterine cavity.

2. The apparatus of claim 1 wherein the chamber includes a volume of temperature-controlled liquid in a housing that has a volume-monitoring window.

3. The apparatus of claim 1 further comprising means to regulate and monitor pressure and temperature of heated fluid and further provides a closed hydraulic system to reliably transfer pressures to ensure balloon filling and emptying.

## Patentansprüche

1. Vorrichtung für die thermische Ablation von Gewebe in einer Gebärmutter, wobei die Vorrichtung folgendes aufweist:
(a) einen Katheter mit einem proximalen Ende und einem distalen Ende;
(b) vorgeformte, aufblasbare Ballons in zwei Größen (für Gebärmuttern mit und ohne Geburtserfahrung), die individuell an dem distalen Ende des Katheters angebracht werden und sich an die Form der Gebärmutter anpassen;
(c) ein mit dem distalen Ende des Katheters verbundenes Ballonentfaltungssystem, das über eine mechanische und/oder hydraulische Einrichtung entfaltet wird, die wiederum mit dem Ballon zusammenwirkt und den vorgeformten Ballon entfaltet;
(d) eine Ballonaufblaseinrichtung zum Drücken von Aufblasfluid durch das Element mit Fassungsvermögen und den Katheter hindurch sowie in den Ballon hinein;
(e) eine Ballonentleerungseinrichtung zum Abführen von Aufblasfluid aus dem Ballon, dem Katheter sowie dem Element mit Fassungsvermögen;
(f) eine Heizeinrichtung zum Erwärmen des Aufblasfluids auf eine erhöhte Temperatur von über 104,4 °C (220 °F);
(g) eine Kammer zum Bereitstellen eines Elements mit Fassungsvermögen zur Ermöglichung einer zuverlässigen Drücküberwachung, Dampffreisetzung, Dampfabscheidung und eines ausreichenden Volumens zum Verhindern eines Temperaturabfalls aufgrund von Wärmeverlust; und
wobei der Katheter eine Längsachse aufweist und wobei die mechanische Ballonentfaltungseinrichtung Entfaltungselemente beinhaltet, die zwei Stellungen aufweisen, - eine zusammengefaftete Stellung, in der die Entfaltungselemente im wesentlichen parallel zu der Längsachse sind, sowie eine ausgefahrene Stellung, in der sich die Elemente rechtwinklig zu der Längsachse des Katheters erstrecken und an dem Ende des Katheters angebracht sind, wodurch eine korrekte Ballonentfaltung und Anpassung an das Innere der Gebärmutterhöhle gewährleistet sind.

2. Vorrichtung nach Anspruch 1,
wobei die Kammer ein Volumen einer temperaturgesteuerten Flüssigkeit in einem Gehäuse enthält, das ein Volumenüberwachungsfenster aufweist.

3. Vorrichtung nach Anspruch 1,
weiterhin mit einer Einrichtung zum Regulieren und Überwachen des Drucks und der Temperatur des erwärmten Fluids unter weiterer Bereitstellung eines geschlossenen Hydrauliksystems zum zuverlässigen Übertragen von Druck, um das Füllen und Entleeren des Ballons zu gewährleisten.

## Revendications

1. Appareil pour l'ablation thermique d'un tissu dans un utérus, comprenant :
(a) un cathéter ayant une extrémité proximale et une extrémité distale ;
(b) des ballonnets gonflables préfaçonnés de deux dimensions (pour des utérus pares et nullipares) fixés individuellement à l'extrémité distale du cathéter, qui épouseront la forme de l'utérus ;
(c) un système de déploiement de ballonnet connecté à l'extrémité distale du cathéter, qui est déployé par un moyen mécanique et/ou hydraulique qui vient alors en prise avec le ballonnet et qui déploie le ballonnet préfaçonné ;
(d) un moyen de gonflage de ballonnet pour entraîner un fluide de gonflage à travers l'élément capacitif, le cathéter et dans le ballonnet ;
(e) un moyen de dégonflage de ballonnet pour décharger le fluide de gonflage du ballonnet, du cathéter et de l'élément capacitif ;
(f) un moyen de chauffage pour chauffer le fluide de gonflage à une température élevée supérieure à 104,4°C (220° F) ;
(g) une chambre pour fournir un élément capacitif permettant un contrôle fiable de pression, une évacuation de vapeur, une séparation de vapeur et un volume suffisant pour empêcher un abaissement de température par perte de chaleur ; et
dans lequel le cathéter a un axe longitudinal et dans lequel le moyen mécanique de déploiement de ballonnet comprend des éléments de déploiement qui possèdent deux positions - une position contractée dans laquelle les éléments de déploiement sont pratiquement parallèles à l'axe longitudinal et une position étendue dans laquelle les éléments s'étendent perpendiculairement à l'axe longitudinal du cathéter et sont fixés à l'extrémité du cathéter, ce qui garantit un déploiement convenable du ballonnet et une conformation à l'intérieur de la cavité utérine.

2. Appareil suivant la revendication 1, dans lequel la chambre comprend un volume de liquide à température régulée dans un boîtier qui possède une fenêtre de contrôle de volume.

3. Appareil suivant la revendication 1, comprenant en outre un moyen pour réguler et contrôler la pression et la température du fluide chauffé et qui comprend en outre un système hydraulique clos pour transférer de manière fiable des pressions afin de garantir le remplissage et le vidage des ballonnets.
